(19) Europäisches Patentamt
European Patent Office
Office européen des brevets

(11) Publication number: 0 095 133
B1

# (12) EUROPEAN PATENT SPECIFICATION

(45) Date of publication of the patent specification: 21.08.85

(51) Int. Cl.⁴: C 07 C 33/02, C 07 C 29/76, C 07 C 69/145, C 07 C 67/56

(21) Application number: 83104874.9

(22) Date of filing: 17.05.83

(54) **Preparation of polyprenyl compounds or mixtures thereof.**

(30) Priority: 21.05.82 JP 87049/82
27.05.82 JP 90887/82

(43) Date of publication of application: 30.11.83 Bulletin 83/48

(45) Publication of the grant of the patent: 21.08.85 Bulletin 85/34

(84) Designated Contracting States: CH DE FR GB IT LI NL

(56) References cited:
Chemical Abstracts vol. 71, no. 19, 10 November 1969, Columbus, Ohio, USA B. TOMITA et al. "Terpenoids. XXII. Chemotaxonomy of the Cupressaceae. 1. The composition of the essential oil from the wood of Thuja koraiensis", page 89, column 2, abstract no. 88431r
Chemical Abstracts vol. 87, no. 15, 10 October 1977, Columbus, Ohio, USA R. CAPUTO et al. "Diterpenoids of Araucariaceae. VII. Minor diterpenes from the resin of Araucaria imbricata", page 338, column 2, abstract no. 114628n
JOURNAL OF THE CHEMICAL SOCIETY PERKIN I, no. 10, 1980, London T. SUGA et al. "Structure and biosynthesis of cleomeprenols from the leaves of cleome spinosa", pages 2098-2104
Chemical Abstracts vol. 82, no. 15, 14 April 1975, Columbus, Ohio, USA K. HANNUS et al. "Polyisoprenols in Pinus sylvestris needles", page 223, column 2, abstract no. 95272v
TETRAHEDRON LETTERS, vol. 50, 1978, London Z. HORII et al. "Ulmoprenol. A new type C30-polyprenoids from eucommia ulmonides, Oliver", pages 5015-5016
Patent Abstracts of Japan vol. 6, no. 177, 11 September 1982

(73) Proprietor: KURARAY CO., LTD., 1621 Sakazu, Kurashiki-City Okayama Prefecture (JP)

(72) Inventor: Ibata, Koichi, c/o Kuraray Ranpu-ryo 1652, Sakazu, Kurashiki City Okayama Prefecture (JP)
Inventor: Takagawa, Tetsuo, 160-4, Bakuro-Cho, Kurashiki City Okayama Prefecture (JP)
Inventor: Okada, Masufumi, 1625, Sakazu, Kurashiki City Okayama Prefecture (JP)
Inventor: Mizuno, Masao, 335-18, Shimosho, Kurashiki City Okayama Prefecture (JP)
Inventor: Nishida, Takashi, 3-9, Kurashikihaitsu, Kurashiki City Okayama Prefecture (JP)

(74) Representative: Vossius Vossius Tauchner Heunemann Rauh, Siebertstrasse 4 P.O. Box 86 07 67, D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



BUNDESDRUCKEREI BERLIN

**Description**

This invention relates to a method of preparing polyprenyl compounds or mixtures thereof. More particularly, the invention relates to a method of preparing polyprenols or esters thereof, or mixtures thereof, which have trans and cis configurations similar to those of dolichol and are useful as advantageous starting materials for the synthesis of dolichol.

Dolichol was first isolated in 1960 from the human kidney and such animal organs as ox kidney, pig kidney, pig heart, pig liver and rat liver by J. F. Pennock et al. [see Nature (London), 186, 470 (1960)]. Later, it was elucidated that dolichol is a mixture of polyprenol homologs having the general formula:

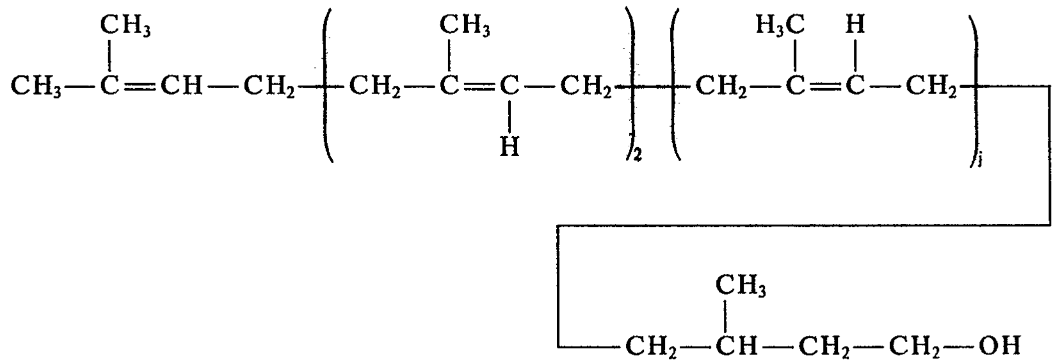

(A)

wherein

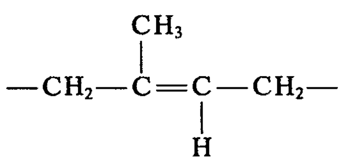

represents a trans-isoprene unit, and

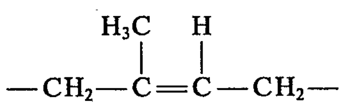

represents a cis-isoprene unit (the same definitions being consistently applied throughout the present text. The number of cis-isoprene units is generally between 12 to 18 and the three homologs in which j is 14, 15 and 16 are present in major proportions [R. W. Keenan et al., Biochemical Journal, 165, 405 (1977)]. It is also known that dolichol is widely distributed in mammals, and performs a very important function in sustaining the lives of organisms. For example, J. B. Harford et al. demonstrated by in vitro tests using the calf or pig brain white matter that exogenous dolichol enhances incorporation of carbohydrates such as mannose into lipid, and consequently increases the formation of glycoproteins which are important for maintaining the lives of organisms [Biochemical and Biophysical Research Communications, 76, 1036 (1977)]. Since the effect of dolichol to incorporate carbohydrates into lipid is remarkable in mature animals as compared with those in the actively growing stage, the action of dolichol has attracted attention for its possible retarding or prevention of aging.

R. W. Keenan et al. state that it is important for organisms which rapidly keep growing, for example, those in the infant stage, to take dolichol extraneously such as to supplement the dolichol produced by biosynthesis within their own body [Archives of Biochemistry and Biophysics, 179, 634 (1977)].

Akamatsu et al. determined the quantity of dolichol phosphate in the regenerated liver of a rat and found that the quantity determined is much smaller than that in normal liver and the function of the liver tissues to synthesize glycoproteins is drastically reduced and that the addition of exogenous dolichol phosphate improves the reduced function of glycoprotein synthesis (reported at the 1981 Conference of the Japanese Society of Biochemistry).

Thus, dolichol is a very important substance for living organisms, and it is strongly desired to develop its use as a medicine or as an intermediate for the production of medicines, cosmetics, etc.

However, since dolichol has hitherto been difficult to obtain, sufficient research work has been impossible For example, only about 0.6 g at most of dolichol can be obtained from 10 kg of pig liver through complicated separating procedures [see J. Burgos et al., Biochemical Journal, 88, 470 (1963)].

On the other hand, it is extremely difficult by present day techniques of organic synthesis to produce dolichol by a wholly synthetic process, as can be seen in the light of the complex and unique molecular structure thereof.

It is to date known that polyprenol compounds can be extracted from various plants, and so far, the

following polyprenols have been successfully extracted.

(1) Solanesol

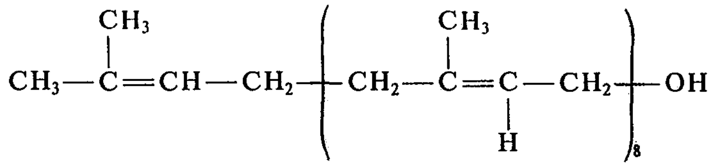

(B)

(2) Ficaprenols

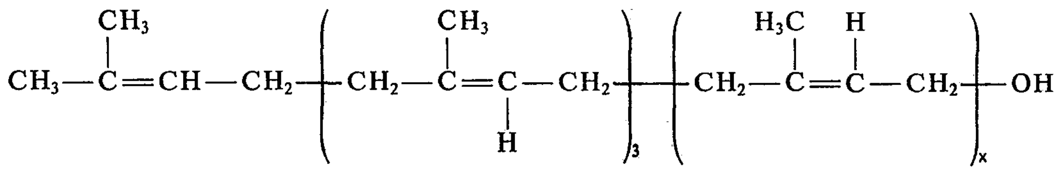

(C)

x=5—9

(3) Betulaprenols

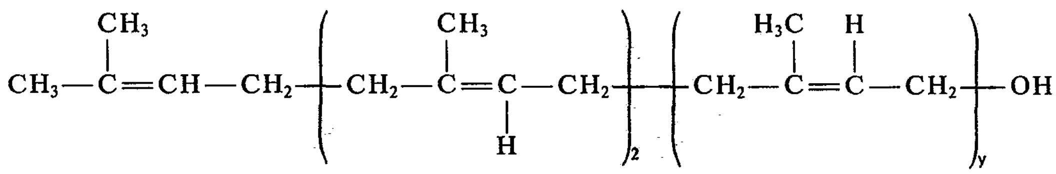

(D)

y=4—6

Betulaprenols have a structure similar to dolichols in that a group of two trans-isoprene units is connected to the omega-terminal isoprene unit and then a group of cis-isoprene units follows. However, the betulaprenols so far known contain up to six cis-isoprene units at most, and in order to synthesize dolichols containing homologs having 14, 15 and 16 cis-isoprene units respectively as major components from these betulaprenols, it is necessary to link at least 8 isoprene units while maintaining them in cis-form. This procedure is almost impossible by the present-day organic synthetic techniques.

K. Hannus et al. reported that a polyisoprenyl fraction in an amount of about 1% dry weight was isolated from the needles of Pinus sylvestris and the fraction consisted of polyisoprenyl acetates with 10 to 19 isoprene units predominantly in the cis-configuration [Phytochemistry, 13, 2563, (1974)]. However, their report does not explain the details of the trans and cis configurations in said polyprenyl acetates. Furthermore, according to a report of D. F. Zinkel et al., a $C_{90}$ polyprenol containing 18 isoprene units or a homologous series of polyprenols averaging 18 isoprene units is present in Pinus strobus needle extracts [cf. Phytochemistry, 11, 3387 (1972)]. However, this report does not contain any detailed analysis of the trans and cis configurations in said polyprenol.

Some of the present inventors, together with their colleagues, previously found that extraction of the leaves of Ginkgo biloba and Cedrus deodara followed by an adequate separation procedure, such as chromatography or fractional dissolution, if necessary following hydrolysis, gives a polyprenyl fraction composed of a mixture of polyprenols and/or acetates thereof which contain 14—22 isoprene units in quite the same trans/cis configurations as in mammalian dolichols and that said polyprenyl fraction is very similar in the chain length distribution of polyprenyl homologs to mammalian dolichols, the only difference being the absence in said fraction of the alpha-terminal saturated isoprene unit and that said polyprenyl fraction, if desired, can be separated relatively easily into the individual constituent polyprenyl homologs (each being homogeneous with respect to the number of isoprene units), and proposed a method of producing dolichols or precursors thereof which comprises reacting such polyprenyl compound or fraction or a reactive derivative thereof with a Grignard reagent or lithium compound derived from a 4-hydroxy-2-methylbutyl halide or a functional precursor thereof (EP 0 054 753 Al published on June 30, 1982).

However, the recovery of the above-mentioned polyprenyl fraction from the leaves of Ginkgo biloba and Cedrus deodara is limited regionally as well as quantitatively from the natural resource viewpoint.

Accordingly, an object of the invention is to provide a method of obtaining polyprenyl compounds or mixtures thereof, which are usable as advantageous starting materials in the synthesis of dolichol, from plants other than the above-mentioned Ginkgo biloba and Cedrus deodara.

Other objects of the invention will become apparent from the following detailed description.

Surprisingly, it has now been found that a polyprenyl fraction relatively rich in polyprenols and/or

polyprenyl acetates having the same trans and cis configurations as those of dolichol and containing 14 to 16 cis-isoprene units can be obtained from the leaves of the plants belonging to the family Cupressaceae, Taxodiaceae, Sciadopitydaceae, Podocarpaceae, Cephalotaxaceae, Araucariaceae or Taxaceae, that, if desired, said polyprenyl fraction can be separated relatively easily into individual constituent polyprenyl homologs, and that said polyprenyl fraction and the individual polyprenyl homologs isolated therefrom are suited for use as intermediates in the synthesis of mammalian dolichol.

Thus, the present invention provides a method of preparing polyprenyl compounds or mixtures thereof represented by the general formula

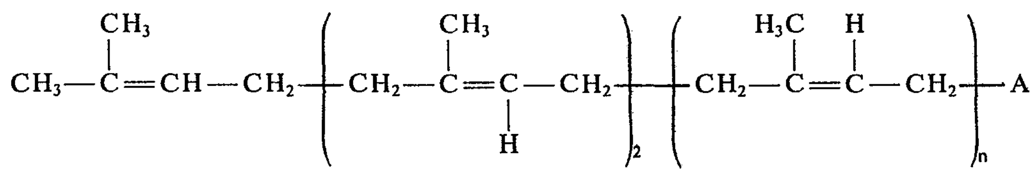 (I)

wherein A is a hydroxyl group or an acyloxy group,

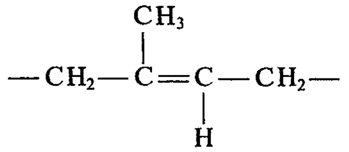

represents a trans-isoprene unit,

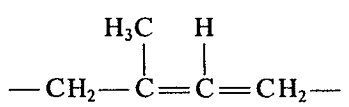

represents a cis-isoprene unit and n is an integer of 10 to 22, preferably 12—18, which comprises extracting leaves of a plant belonging to the family Cupressaceae, Taxodiaceae, Sciadopitydaceae, Podocarpaceae, Cephalotaxaceae, Araucariaceae or Taxaceae with an organic solvent and isolating a polyprenyl compound or a mixture of polyprenyl compounds from the extract, if necessary following or followed by subjecting the extract or the isolated compound or mixture to one or more reactions selected from among hydrolysis, esterification and transesterification.

The plants of the families Cupressaceae, Taxodiaceae, Sciadopitydaceae, Podocarpaceae, Cephalotaxaceae, Araucariaceae and Taxaceae, which are used as the starting materials in the practice of the present invention, are plants belonging to the order Coniferales of the class Coniferopsida of the sub-division Gymnospermae of the division Spermatophyta. (Hereinafter said plants are sometimes referred to as »starting plants« for short.)

The plants of the family Cupressaceae are widely distributed on the earth and said family is divided into 19 genera and about 130 species. Examples of the plants of the family Cupressaceae, which can be used as the starting plants, include Calocedrus decurrens, Chamaecyparis obtusa (Japanese cypress), Chamaecyparis nootkatensis, Chamaecyparis lawsoniana, Cupressus torulosa, Cupressus sempervirens, Cupressus funebris, Juniperus chinensis (Chinese juniper), Juniperus communis (common juniper), Juniperus rigida, Juniperus horizontalis, Juniperus oxycedrus, Juniperus ashei, Juniperus occidentalis, Juniperus virginiana, Thuja standishii, Thuja occidentalis, Thuja plicata and Thujopsis dolabrata var. dolabrata. Among them, plants of the genus Chamaecyparis, such as Chamaecyparis obtusa, and plants of the genus Juniperus, such as Juniperus chinensis and Juniperus rigida, are preferably used.

The plants of the family Taxodiaceae are widely distributed in the temperate zone in the northern hemisphere, especially in China, and said family is divided into 9 genera and 15 species. Examples of the plants of the family Taxodiaceae, which can be used as the starting plants, include Cryptomeria japonica (Japanese cedar), Cunninghamia lanceolata, Glyptostrobus pensilis, Metasequoia glyptostroboides, Sequoia sempervirens, Sequoiadendron giganteum, Taiwania cryptomerioides and Taxodium distichum. Among them preferably used are plants of the genus Cryptomeria, such as Cryptomeria japonica, and the plant of the genus Metasequoia, namely Metasequoia glyptostroboides.

The family Sciadopitydaceae comprises only one genus, Sciadopitys, which in turn includes only one species, namely Sciadopitys verticillata (parasol fir). This plant is found mainly in Japan.

The plants of the family Podocarpaceae are distributed mainly in the temperate zone in the southern hemisphere as well as in the subtropical zones, and said family is divided into 11 genera and about 125 species. Examples of the plants of the family Podocarpaceae, which can be used as the starting plants, include Podocarpus macrophylla (podocarp), Podocarpus nagi, Podocarpus nivalis, Podocarpus hallii, Podocarpus lawrencii, Podocarpus neriifolius and Phyllocladus hypophyllus. Preferably among them

are plants of the genus Podocarpus, such as Podocarpus macrophylla and Podocarpus nagi.

The plants of the family Cephalotaxaceae are distributed mainly in East Asia. Said family comprises only one genus, Cephalotaxus, which is divided into 6 species, and plants of this genus, such as Cephalotaxus harringtonia subsp. nana, are preferably used.

The plants of the family Araucariaceae are distributed mainly in the subtropical zones and in the temperate zone in the southern hemisphere. This family is divided into two genera, namely Agathis and Araucaria, and 38 species. Examples of the plants of the family Araucariaceae, which can be used as the starting plants, include Agathis australis, Agathis dammara, Agathis labillardieri, Araucaria brasiliana, Araucaria araucana, Araucaria angustifolia, Araucaria cunninghamii and Araucaria heterophylla. Among them, Araucaria brasiliana is preferably used, for instance.

The plants of family Taxaceae are distributed widely in the northern hemisphere and said family is divided into 5 genera and 20 species. As typical examples of the plants of this family, there may be mentioned Taxus cuspidata, Taxus baccata (yew) and Torreya nucifera (Japanese nutmeg). Among them, plants of the genus Taxus, such as Taxus cuspidata, and plants of the genus Torreya, such as Torreya nucifera, are preferably used.

If desired, mixed leaves from two or more of the above-mentioned starting plants may be subjected to extraction in practicing the present invention.

The leaves to be treated according to this invention may be used undreid or after drying. Generally, the dried leaves are preferred. The degree of drying of the leaves should advantageously correspond to a water content, based on the weight of the dried leaves, of less than about 30%, preferably less than about 10%. Preferably, the leaves are extracted after crushing. This increases the area of contact with the extracting solvent, and results in an increased efficiency of extraction.

The polyprenyl homologs of formula (I) are contained in the leaves of the starting plants in fairly high concentrations generally in the form of a free alcohol and/or acetic acid ester. In order to extract the polyprenyl homologs from the leaves of these plants effectively, the use of oil-soluble organic solvents capable of well dissolving the polyprenyl homologs is convenient.

Susitable oil-soluble organic solvents that can be used in practicing this invention have a dielectric constant ($\varepsilon$) of not more than 32.7, preferably not more than 25.0, more preferably not more than 20.7. Specifically, solvents such as exemplified below are used either singly or as a mixture of two or more.

(a) Hydrocarbons such as petroleum ether, pentane, hexane, heptane, benzene, toluene and xylene.
(b) Halogenated hydrocarbons such as chloroform, methylene chloride, carbon tetrachloride, tetrachloroethane, perchloroethylene and trichloroethylene.
(c) Esters such as methyl acetate, ethyl acetate and ethyl propionate.
(d) Ethers such as diethyl ether, diisopropyl ether, tetrahydrofuran and dioxane.
(e) Ketones such as acetone, methyl ethyl ketone, diethyl ketone and diisopropyl ketone.
(f) Alcohols such as methyl alcohol, ethyl alcohol, propyl alcohol and butyl alcohol.

The solvent should desirably be capable of extracting the desired polyprenyl compounds of formula (I) selectively with a high efficiency, while permitting minimization of extraction of other substances. From this standpoint, the hydrocarbons, halogenated hydrocarbons, esters, and ethers having low polarity such as diethyl ether and diisopropyl ether, and ketones are especially suitable among the above exemplified solvents.

The amount of the extracting solvent is not critical, and can be varied widely depending upon the type of the solvent, the type or condition of the leaves to be extracted, etc. Generally, it is advantageous that the solvent is used in an amount of about 1 to about 100 parts by weight, preferably 5 to 50 parts by weight, more preferably 10 to 30 parts by weight, per part (based on the dry weight) of the leaves.

The extraction can be carried out by dipping the leaves in the solvent, and if required, stirring the mixture continuously or intermittently. The temperature during the extraction is not critical, but can be varied widely depending upon the extraction conditions such as the type or amount of the solvent used. Generally, the extraction temperature is from about 0°C to the refluxing temperature of the solvent. Usually, room temperature suffices. Under these conditions, the extraction should advantageously be carried out for a period of 1 to 10 days.

After the extracting treatment, the leaves and other solid components are removed from the dipping solution and if required, the solvent is removed to give a concentrate. The extract is subjected to a separating step consisting of one or more of chromatography, fractional dissolution, fractional refrigerating precipitation and molecular distillation, whereby the desired polyprenyl fraction is recovered.

In the separating step, the formation of the fraction of polyprenyl compounds can be confirmed by determining whether a spot exists at an Rf value of from 0.18 to 0.25 [when A in formula (I) represents a hydroxyl group] and/or from 0.50 to 0.55 [when A in formula (I) represents an acetyloxy group] in thin-layer chromatography which is carried out on a TLC plate of Merck Co. precoated with silica gel $60F_{254}$ to a layer thickness of 0.25 mm with a mixture of n-hexane and ethyl acetate in a volume ratio of 9 : 1 as a developing solvent (developed 10 cm) under such conditions that solanesyl acetate as a standard substance shows an Rf value of from 0.40 to 0.45 in the thin-layer chromatography. It should

be understood that in the following description, the Rf values of thin-layer chromatography denote those which are measured under the aforesaid conditions unless otherwise specified.

The operations of the chromatography, fractional dissolution, fractional refrigerating precipitation and molecular distillation used in the step of separating the aforesaid extract are known per se, and in the present invention, too, these methods can be carried out in accordance with known procedures. For the details of these methods, literature references will be cited in lieu of describing them at length. Only those items which need special care will be described below.

(A) Chromatography

For details, reference may be made to H. Heftman, »Chromatography«, Reinhold Publishing Co., New York (1961)

When the amount of the extract is small, thin-layer chromatography or liquid chromatography is suitable. For treatment of a large amount of the extract, column chromatography is suitable.

Examples of suitable chromatographic carriers are silica gel, alumina, Florisil, Celite, activated carbon, and cellulose. Silica gel is especially preferred.

Examples of the developing solvent usable in the separating operation on a silica gel column include hexane/ethyl acetate (volume ratio from 98 : 2 to 80 : 20), hexane diisopropyl ether (volume ratio from 95 : 5 to 80 : 20), petroleum ether/methyl acetate (volume ratio from 98 : 2 to 80 : 20), petroleum ether/isopropyl alcohol (volume ratio from 99 : 1 to 90 : 10), benzene/diethyl ether (volume ratio from 95 : 5 to 80 : 20), benzene/ethyl acetate (volume ratio from 98 : 2 to 80 : 20), chloroform and methylene chloride.

(B) Fractional dissolution

For details, reference may be made to L. C. Craig, »Technique of Organic Chemistry«, Vol. 3, Interscience (1951)

The polyprenyl compounds of formula (I) are easily soluble in non-polar solvents such as pentane and hexane, and are sparingly soluble in polar solvents such as methanol or water. Hence, the polyprenyl compounds of formula (I) can be purified by the fractional dissolving method utilizing differences in solubility in these solvents. For example, a crude product such as a concentrate of the extract is dissolved in the aforesaid nonpolar solvent, and then washed with a polar solvent which is immiscible with the nonpolar solvent, whereby impurities easily soluble in the polar solvent can be drastically removed. Suitable nonpolar solvents for use in this method are, for example, hydrocarbons such as petroleum ether, pentane, hexane, heptane, benzene and toluene and halogenated hydrocarbons such as methylene chloride and chloroform. Suitable polar solvents immiscible with such nonpolar solvents are, for example, water and methanol.

(C) Fractional refrigerating precipitation

For details, reference may be made to E. W. Berg, »Physical and Chemical Methods of Separation«, Chapters 14 and 15, McGraw-Hill, New York (1963)

The polyprenyl compounds of formula (I) solidify at about −10°C or less. Hence, the polyprenyl compounds of formula (I) can be purified by allowing the extract to stand at a temperature of not more than −10°C, preferably −15 to −30°C, to solidify the desired compounds, and removing the impurities which do not solidify at these temperatures by a solid-liquid separating technique. The polyprenyl compounds, however, do not have good crystallinity and become a waxy solid. Accordingly, they are difficult to purify completely by this method alone. Preferably, therefore, this method is used in combination with another purifying method possible to remove crystalline impurities contained in the polyprenol by preferentially precipitating them.

(D) Molecular distillation

For details, reference may be made to G. Burrows, »Molecular Distillation«, Clarendon Press, Oxford (1960)

Since the compounds of formula (I) have a high molecular weight, they can be separated from low-molecular-weight impurities by the molecular distillation method. For example, the extract is subjected to molecular distillation at 100 to 250°C under a vacuum of $10^{-3}$ to $10^{-5}$ mm Hg to divide it

into a low-molecular-weight fraction and a high-molecular-weight fraction. The desired compounds are retained in the high-molecular-weight fraction, and the low-molecular-weight impurities can be removed effectively.

When a sufficiently pure polyprenyl fraction cannot be obtained by any of these separating methods, two or more of these separating methods may be used in combination. For example, there can be used a combination of chromatography and fractional dissolution, a combination of chromatography, fractional refrigerating precipitation and fractional dissolution, a combination of chromatography, fractional refrigerating precipitation, fractional dissolution and molecular distillation, a combination of chromatography, molecular distillation and fractional dissolution, a combination of chromatography and molecular distillation, a combination of molecular distillation and fractional dissolution, and a combination of molecular distillation, fractional dissolution and fractional refrigerating precipitation.

As a result of the separating step, a fraction having an Rf value of from 0.18 to 0.25, and/or from 0.50 to 0.55 in thin-layer chromatography can be isolated and recovered. The fraction having an Rf value of 0.18 to 0.25 consists essentially of a mixture of homologs of formula (I) in which A represents a hydroxyl group, and the fraction having an Rf value of from 0.50 to 0.55 consists essentially of homologs of formula (I) in which A represents an acetyloxy group.

The polyprenyl fraction thus obtained can, if necessary, be subjected to liquid chromatography or column chromatography, for instance, so that a fraction consisting of those homologs that are adequate with respect to the number of cis-isoprene units can be obtained with other homologs being cut off. Said fraction can also be separated into the individual homologs by subjecting it further, for example, to high performance liquid chromatography.

Prior to subjecting the extract to the aforesaid separating operation, the extract may be hydrolyzed as required to convert homologs of formula (I) in which A represents an acetyloxy group into homologs of formula (I) in which A represents a hydroxyl group. Of course, the hydrolysis may also be carried out on a fraction having an Rf value of 0.50 to 0.55 obtained by the separating operation. The hydrolysis can be performed by any usual methods known for the hydrolysis of known fatty acid esters. For example, about 5 to about 50 parts by weight of the extract or the fraction is added to 100 parts by weight of a solution of sodium hydroxide or potassium hydroxide in aqueous methanol or ethanol (the alkali metal hydroxide concentration being preferably about 0.1 to 30% by weight), and the reaction is effected at about 25 to 90° C for about 0.5 to 5 hours.

Referring to the polyprenyl fraction separated and recovered by the method described hereinabove, the fraction having an Rf value of 0.18 to 0.25 consists essentially of a mixture of polyprenol homologs of formula (I) in which A represents a hydroxyl group, and the fraction having an Rf value of 0.50 to 0.55 consists essentially of a mixture of polyprenyl acetate homologs of formula (I) in which A represents an acetyloxy group. The ratio of the former to the latter in the extract is generally in the range of from about 20 : 1 to about 1 : 1000. The distribution pattern for the polyprenol or polyprenyl acetate homologs in each fraction varies to some extent depending on the kind of the starting plant. The distribution pattern remains almost the same for starting plants of the same species without depending on such factors as the age of the leaves used as the raw material and the time and place of collection of the leaves.

In deriving mammalian dolichols from a polyprenyl fraction prepared by the method of the present invention, said fraction may be used as such or, if necessary, said fraction may be deprived of those homologs which are unnecessary for the synthesis of dolichols equivalent in the number of cis-isoprene units to natural dolichols and then subjected to dolichol synthesis or said fraction may be separated into individual constituent polyprenyl compounds for subjecting adequate ones alone to dolichol synthesis. Furthermore, said fraction or each isolated polyprenyl compound may be subjected to hydrolysis, esterification or transesterification or a combination of two or more of these reactions depending on whether it is in the form of free alcohol or acetate, for conversion of polyprenols to polyprenyl esters or conversion of polyprenyl acetates to other polyprenyl esters of general formula (I) or to polyprenols.

Examples of the polyprenyl ester of general formula (I) which can be produced by the method of the invention are, when A in general formula (I) is represented by RCOO—, those esters in which R is a hydrogen atom, an alkyl group containing 1 to 18 carbon atoms (e. g. methyl, ethyl, n-propyl, isopropyl, n-butyl, sec-butyl, isobutyl, tert-butyl, n-penthyl, isoamyl, n-hexyl, n-octyl, n-decyl, n-undecyl, n-dodecyl, stearyl), an alkenyl group containing 2 to 18 carbon atoms (e. g. 3-butenyl, 3-pentenyl, 4-pentenyl, geranyl, geranylmethyl, farnesyl, farnesylmethyl), a cycloalkyl group containing 5 to 7 carbon atoms (e. g. cyclopentyl, cyclohexyl, methylcyclohexyl, cycloheptyl), an aryl group containing 6 to 10 carbon atoms (e. g. phenyl, tolyl, xylyl, naphthyl), an aralkyl group containing 7 to 11 carbon atoms (e. g. benzyl, phenethyl, methylbenzyl, dimethylbenzyl, alpha- or beta-naphthylmethyl) or a methyl group substituted by 1 to 3 fluorine or chlorine atoms (e. g. monofluoromethyl, trifluoromethyl, monochloromethyl, dichloromethyl). The esterification or transesterification of a polyprenol or polyprenol mixture represented by general formula (I) for the production of the above-mentioned polyprenyl ester or polyprenyl ester mixture can be carried out in the same manner and under the same conditions as in the per se known esterification or transesterification reactions for the production of esters. Thus, for example, the esterification can be easily attained by mixing a polyprenol or polyprenol mixture

represented by general formula (I) with a carboxylic acid as desired, such as formic acid, acetic acid, monofluoroacetic acid, monochloroacetic acid, trifluoroacetic acid, propionic acid, butyric acid, caproic acid, caprylic acid, capric acid, lauric acid, palmitic acid, stearic acid, oleic acid, linolic acid, linoleic acid, farnesylic acid, farnesylacetic acid, benzoic acid, 3,5-dimethylbenzoic acid or 4-ethylbenzoic acid, or an acid halide or acid anhydride thereof in the presence or absence of an esterification catalyst and of a solvent, if necessary with or followed by heating and/or stirring. In a preferred embodiment, the above polyprenol or polyprenol mixture is dissolved in a solvent such as hexane, benzene, methylene chloride, chloroform or diethyl ether, the acid halide mentioned above is added in an amount of 1 to 5 moles per mole of the polyprenol and the resulting mixture is stirred in the presence of 1 to 5 moles of pyridine per mole of the polyprenol at an adequate temperature in the range of from room temperature to the boiling point of the solvent for 1 to 24 hours.

The transesterification is carried out by reacting a polyprenol or polyprenol mixture represented by general formula (I) with a lower alkyl ester (e. g. methyl ester, ethyl ester) of a carboxylic acid as desired (for instance, one of the carboxylic acids mentioned above) optionally in the presence of a transesterification catalyst. In a preferred embodiment, the above polyprenol or polyprenol mixture and a desired carboxylic acid ester are dissolved in a solvent such as benzene, toluene or xylene, sodium hydride is added in an amount of 0.01 to 0.1 mole per mole of the polyprenol and the resulting mixture is refluxed for a period of 2 hours to 5 days.

The polyprenyl compounds or mixtures thereof represented by general formula (I) as obtained by the method of the invention are easily converted to mammalian dolichols, for example by the following routes:

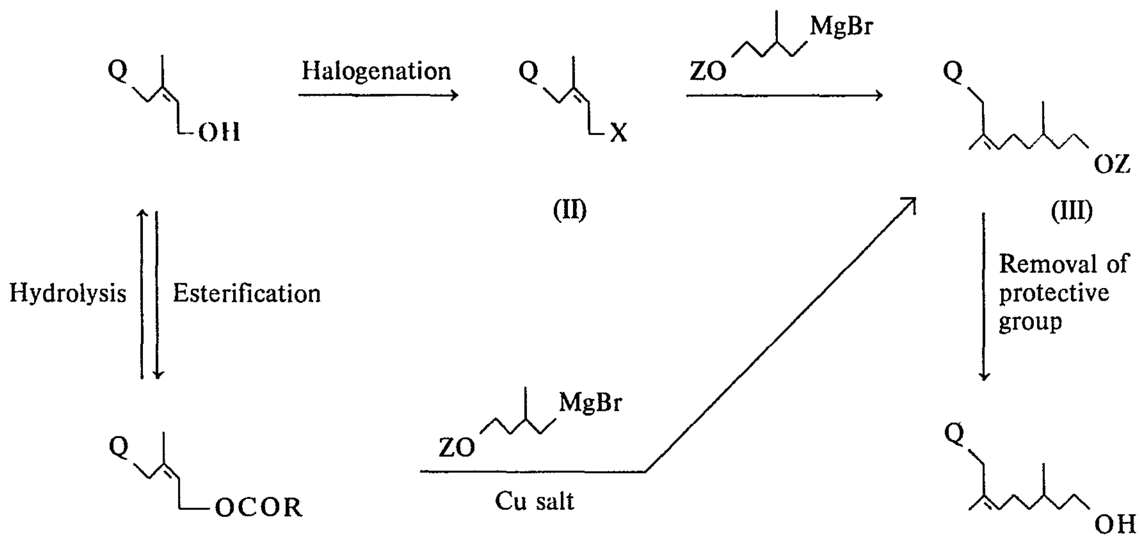


In the above schema, X is a halogen atom, preferably Cl or Br, Z is a hydroxyl-protecting group, such as, for example, tetrahydropyranyl, methoxymethyl or benzyl, OCOR is an acyloxy group and Q is a group of the formula

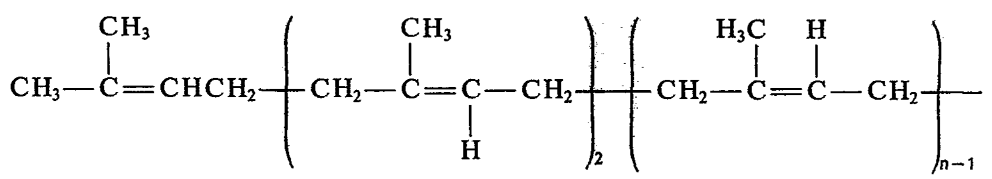
(IV)

wherein n is as defined for general formula (I).

The polyprenyl compounds and mixtures thereof obtainable by the method of the present invention are also useful, for instance, as cosmetic bases or ointment bases or as the raw materials for the production thereof.

The following nonlimitative examples illustrate the invention in more detail. In the examples and reference example, the infrared (IR) analysis was always performed by the liquid film method, and the nuclear magnetic resonance (NMR) analysis was conducted in $CDCl_3$ with tetramethylsilane (TMS) as an internal standard. The field desorption mass spectrometry (FD-MASS) analysis data values are values corrected on the $^1H$, $^{12}C$, $^{16}O$ and $^{79}Br$ basis.

## Example 1

Leaves (10 kg, undried weight) of Cryptomeria japonica as collected late June in Kurashiki City were

air-dried at about 50° C for 24 hours and then immersed in 80 liters of chloroform at room temperature (abough 20° C) for 1 week for extraction. The chloroform was distilled off from the extract, 5 liters of petroleum ether was added to the concentrate thus obtained, the insoluble matter was filtered off, and the filtrate was concentrated and subjected to silica gel chromatography using chloroform as the developing solvent. There was thus obtained about 25 g of an oil as a fraction (crude polyprenyl fraction) showing Rf values of 0.52 and 0.19 in thin layer chromatography [Merck thin layer chromatographic plate coated with silica gel $60F_{254}$ to a layer thickness of 0.25 mm; developing solvent: n-hexane-ethyl acetate = 9 : 1 (by volume); developed 10 cm]. In the above thin layer chromatography, solanesyl acetate gave an Rf value of 0.41. To this oil was added about 300 ml of acetone for dissolution of acetone-soluble components, the mixture was filtered, and the filtrate was concentrated. The thus-obtained oil was heated with 300 ml of methanol, 30 ml of water and 20 g of sodium hydroxide at 65° C for 2 hours, then the methanol was distilled off, diethyl ether (400 ml) was added to the residue for extraction, the ether layer was washed five times with about 100 ml of water and then dried over anhydrous sodium sulfate, and the solvent was then distilled off to give 19.8 g of an oil. This oil was chromatographed over about 1 kg of silica gel with a n-hexane-diisopropyl ether (90 : 10 by volume) mixture as the eluent to give 17.2 g o an oily fraction showing an Rf value of 0.19 in the above thin layer chromatography. This oily fraction was a polyprenol fraction having a purity of not less than 90%. The molecular weight distribution measured for this oily fraction is shown below. The data indicate the area proportions of individual peaks in the chromatogram obtained by high performance liquid chromatography using a Merck semipreparative high performance liquid chromatography column LiChrosorb RP 18-10 ($C_{18}$ type) with an acetone-methanol (90 : 10) mixture as the eluent and a differential refractometer as the detector.

| Peak No. | Value of n (Number of cis-isoprene units) | Area proportion (%) |
|---|---|---|
| 1 | 11 | 0.6 |
| 2 | 12 | 1.4 |
| 3 | 13 | 6.6 |
| 4 | 14 | 21.5 |
| 5 | 15 | 21.9 |
| 6 | 16 | 5.6 |
| 7 | 17 | 3.1 |
| 8 | 18 | 9.4 |
| 9 | 19 | 16.5 |
| 10 | 20 | 10.2 |
| 11 | 21 | 2.6 |
| 12 | 22 | 0.5 |

Each component was isolated from the above oily fraction by the above high performance liquid chromatography and identified as a polyprenol of general formula (I) wherein A is a hydroxyl group by mass spectrometry, infrared spectroscopy, $^1$H-NMR spectroscopy and $^{13}$C-NMR spectroscopy. Table 1 summarizes the FD-MASS results and $^1$H-NMR $\delta$ values for the respective components and Table 2 summarizes the $^{13}$C-NMR $\delta$ values. In the $^1$H-NMR data, (b) represents a broad signal; (d), a doublet signal; and (t), a triplet signal.

Table 1

| Value n (Number of cis-isoprene units) | FD-MASS (m/e) Found | Calcd. | $^1$H-NMR $\delta$ (ppm) $=C\underline{H}CH_2OH$ | $=C\underline{H}-$ | $-C\underline{H}_2OH$ | $-C\underline{H}_2-$ | 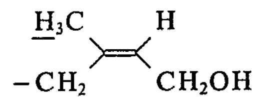 | 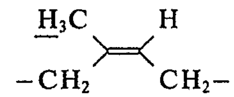 | 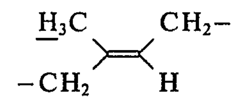 |
|---|---|---|---|---|---|---|---|---|---|
| 11 | 970 | 970 | 5.44 (t) | 5.13 (b) | 4.08 (d) | 2.04 (b) | 1.74 | 1.68 | 1.60 |
| 12 | 1038 | 1038 | 5.44 (t) | 5.12 (b) | 4.08 (d) | 2.04 (b) | 1.74 | 1.68 | 1.60 |
| 13 | 1106 | 1106 | 5.43 (t) | 5.12 (b) | 4.08 (d) | 2.04 (b) | 1.74 | 1.68 | 1.60 |
| 14 | 1174 | 1174 | 5.44 (t) | 5.12 (b) | 4.08 (d) | 2.04 (b) | 1.74 | 1.68 | 1.60 |
| 15 | 1242 | 1242 | 5.44 (t) | 5.13 (b) | 4.08 (d) | 2.04 (b) | 1.74 | 1.68 | 1.60 |
| 16 | 1310 | 1310 | 5.44 (t) | 5.14 (b) | 4.08 (d) | 2.04 (b) | 1.74 | 1.68 | 1.60 |
| 17 | 1378 | 1378 | 5.44 (t) | 5.13 (b) | 4.08 (d) | 2.04 (b) | 1.74 | 1.68 | 1.60 |
| 18 | 1446 | 1446 | 5.43 (t) | 5.13 (b) | 4.08 (d) | 2.05 (b) | 1.74 | 1.68 | 1.60 |
| 19 | 1514 | 1514 | 5.44 (t) | 5.13 (b) | 4.08 (d) | 2.04 (b) | 1.74 | 1.68 | 1.60 |
| 20 | 1582 | 1582 | 5.44 (t) | 5.13 (b) | 4.08 (d) | 2.04 (b) | 1.74 | 1.68 | 1.60 |
| 21 | 1650 | 1650 | 5.44 (t) | 5.13 (b) | 4.08 (d) | 2.04 (b) | 1.74 | 1.68 | 1.60 |
| 22 | 1718 | 1718 | 5.44 (t) | 5.13 (b) | 4.08 (d) | 2.04 (b) | 1.74 | 1.68 | 1.60 |

Table 2

| Value n (Number of cis-isoprene units) | $^{13}$C-NMR δ (ppm) 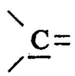 | =CH– | –CH$_2$OH | 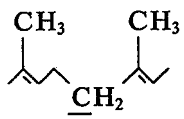 | 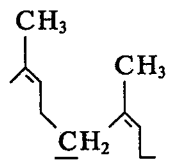 | 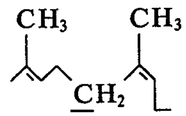 | 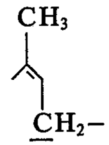 | 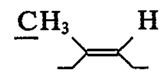 | 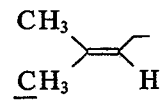 | 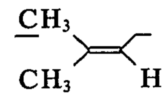 | 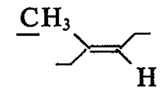 |
|---|---|---|---|---|---|---|---|---|---|---|---|
| 11 | 135.17 | 125.09 | 59.00 | 39.77 | 32.27 | 32.04 | 26.47 | 23.42 | 25.67 | 17.64 | 15.98 |
| 12 | 135.17 | 125.10 | 58.99 | 39.78 | 32.28 | 32.05 | 26.47 | 23.42 | 25.66 | 17.64 | 15.98 |
| 13 | 135.16 | 125.08 | 58.99 | 39.78 | 32.27 | 32.05 | 26.48 | 23.42 | 25.67 | 17.65 | 15.99 |
| 14 | 135.17 | 125.09 | 59.00 | 39.77 | 32.27 | 32.04 | 26.47 | 23.42 | 25.66 | 17.64 | 15.97 |
| 15 | 135.15 | 125.12 | 58.99 | 39.78 | 32.29 | 32.05 | 26.49 | 23.42 | 25.65 | 17.65 | 15.99 |
| 16 | 135.15 | 125.11 | 58.98 | 39.77 | 32.28 | 32.05 | 26.49 | 23.42 | 25.65 | 17.64 | 15.98 |
| 17 | 135.15 | 125.12 | 59.00 | 39.77 | 32.29 | 32.05 | 26.49 | 23.41 | 25.66 | 17.65 | 15.99 |
| 18 | 135.16 | 125.10 | 58.98 | 39.77 | 32.29 | 32.05 | 26.48 | 23.41 | 25.64 | 17.64 | 15.99 |
| 19 | 135.15 | 125.10 | 58.98 | 39.78 | 32.28 | 32.05 | 26.49 | 23.42 | 25.65 | 17.65 | 15.98 |
| 20 | 135.15 | 125.10 | 58.98 | 39.77 | 32.28 | 32.05 | 26.48 | 23.41 | 25.65 | 17.64 | 15.98 |
| 21 | 135.16 | 125.10 | 58.99 | 39.78 | 32.29 | 32.05 | 26.49 | 23.42 | 25.65 | 17.65 | 15.98 |
| 22 | 135.16 | 125.10 | 58.99 | 39.77 | 32.28 | 32.05 | 26.48 | 23.41 | 25.65 | 17.64 | 15.98 |

## Examples 2—7

Proceeding by the procedure of Example 1 under the same conditions as in Example 1 but using leaves of Metasequoia glyptostroboides, Chamaecyparis obtusa, Juniperus chinensis, Juniperus rigida, Taxus cuspidata and Torreya nucifera, respectively, as collected in Okayama Prefecture as the starting materials in place of the leaves of Cryptomeria japonica used in Example 1, polyprenol fractions represented by general formula (I) wherein A is a hydroxyl group were obtained. Each fraction was structurally identified as a polyprenol fraction by mass spectrometry, infrared spectroscopy and NMR analyses of each component isolated from the fraction by high-performance liquid chromatography in the same manner as in Example 1; the analytical results were in substantial agreement with those obtained in Example 1. Table 3 summarizes the yield, molecular weight distribution (chain length distribution; measured in the same manner as in Example 1) and other data for each polyprenol fraction obtained.

Table 3

| | Example No. 2 | 3 | 4 | 5 | 6 | 7 |
|---|---|---|---|---|---|---|
| Starting plant | Metasequoia glyptostroboides | Chamaecyparis obtusa | Juniperus chinensis | Juniperus rigida | Taxus cuspidata | Torreya nucifera |
| Time of collection | Mid-May | Late Oct. | Mid-Nov. | Late May | Early Apr. | Late Nov. |
| Polyprenol Yield (g) | 4.9 | 16.3 | 1.4 | 10.5 | 7.7 | 7.9 |
| Purity (%) | >90 | >90 | >90 | >90 | >90 | >90 |
| Polyprenol molecular weight distribution (Area proportion %) | | | | | | |
| Value of n in formula (I) | | | | | | |
| 11 | 0.1 | 0.5 | 0.2 | 0.6 | 0.5 | 0.6 |
| 12 | 0.1 | 2.9 | 1.0 | 3.2 | 1.0 | 1.4 |
| 13 | 1.5 | 6.7 | 5.4 | 13.1 | 4.6 | 9.3 |
| 14 | 10.1 | 12.1 | 16.3 | 26.5 | 16.5 | 33.4 |
| 15 | 15.7 | 10.8 | 22.8 | 19.8 | 20.8 | 41.5 |
| 16 | 8.3 | 7.0 | 18.4 | 12.0 | 9.8 | 11.7 |
| 17 | 9.0 | 10.6 | 16.5 | 10.2 | 10.0 | 1.7 |
| 18 | 25.2 | 21.5 | 12.0 | 9.1 | 14.3 | 0.5 |
| 19 | 22.6 | 18.8 | 5.7 | 3.9 | 12.7 | — |
| 20 | 6.4 | 7.1 | 1.7 | 1.6 | 6.9 | — |
| 21 | 1.0 | 2.0 | — | — | 2.9 | — |

Example 8

Leaves of Chamaecyparis obtusa (5 kg; undried weight) as collected in Kurashiki City late October were dried with hot air at about 40° C for 12 hours, finely divided in a mixer and then extracted with 40 liters of a petroleum ether-acetone (4 : 1 by volume) mixture at room temperature (about 15° C) for 1 week. The extract was washed with water and dried over anhydrous sodium sulfate, and the solvent was then distilled off to give a residue (about 100 g). To the residue was added one liter of n-hexane for dissolving n-hexane-soluble components, the mixture was filtered, and the filtrate was concentrated and then subjected to silica gel column chromatography using a n-hexane-diethyl ether (95 : 5 by volume) mixture to give about 9.8 g of an oil as a fraction showing an Rf value of 0.50 in thin layer chromatography performed under the same conditions as in Example 1. To this oil was added about 100 ml of acetone for dissolving acetone-soluble components, the mixture was filtered, and the filtrate was chromatographed over about 400 g of silica gel with a n-hexane-diethyl ether (95 : 5 by volume) mixture as the eluent to give 5.5 g of an oil. This oil consisted of polyprenyl acetates and has a purity of not less than 90%. The molecular weight distribution for this oil as determined based on the area proportions of individual peaks in the chromatogram obtained by high performance liquid chromatography using a Waters high performance liquid chromatographic column μBondapak/$C_{18}$ with an acetone-methanol (70 : 30 by volume) mixture as the eluent and a differential refractometer as the detector was as shown below. The data were in good agreement with those for the polyprenol fraction of Example 3 (Table 3) in the ±0.8% range.

| Peak No. | Value of n in formula (I) | Area Proportion (%) |
|---|---|---|
| 1 | 11 | 0.3 |
| 2 | 12 | 3.0 |
| 3 | 13 | 6.8 |
| 4 | 14 | 12.9 |
| 5 | 15 | 10.5 |
| 6 | 16 | 7.6 |
| 7 | 17 | 10.4 |
| 8 | 18 | 21.3 |
| 9 | 19 | 18.8 |
| 10 | 20 | 7.2 |
| 11 | 21 | 1.2 |

The above oil (polyprenyl acetate content: not less than 90%) was subjected to high performance liquid chromatography under the same conditions as in Example 1 to isolate the individual components. Each component was identified as the polyprenyl acetate of general formula (I) wherein A is an acetoxy group by mass spectrometry, infrared spectroscopy, $^{1}H$-NMR spectroscopy and $^{13}C$-NMR spectroscopy. The results of FD-MASS analysis for the respective components were as follows:

| Value of n in formula (I) | FD-MASS analysis Value m/e |
|---|---|
| 11 | 1012 |
| 12 | 1080 |
| 13 | 1148 |
| 14 | 1216 |
| 15 | 1284 |
| 16 | 1352 |
| 17 | 1420 |
| 18 | 1488 |
| 19 | 1556 |
| 20 | 1624 |
| 21 | 1692 |

Each component was hydrolyzed by the same hydrolysis procedure as used in Example 1. The polyprenol obtained gave substantially the same $^1$H-NMR, $^{13}$C-NMR and infrared absorption spectra as those for the corresponding polyprenol having equal n value obtained in Example 1.

Examples 9—14

Following the procedure of Example 8 under the same conditions as in Example 8 but using leaves of Juniperus chinensis, Juniperus rigida, Cryptomeria japonica, Metasequoia glyptostroboides, Taxus cuspidata or Torreya nucifera, respectively, as collected in Okayama Prefecture in place of the leaves of Chamaecyparis obtusa used in Example 8, a polyprenyl acetate fraction represented by general formula (I) wherein A is an acetoxy group was obtained and structurally identified in the same manner as in Example 8. Table 4 summarizes the yield and purity of said polyprenyl acetate fraction. The molecular weight distribution for each polyprenyl acetate fraction was not significantly different from the corresponding polyprenol fraction obtained in Examples 2—7 from the leaves of the same plant species.

Table 4

| | Example No. | | | | | |
|---|---|---|---|---|---|---|
| | 9 | 10 | 11 | 12 | 13 | 14 |
| | Starting plant | | | | | |
| | Juniperus chinensis | Juniperus rigida | Crypto-meria japonica | Metase-quoiaglypto-stroboides | Taxus cuspidata | Torreya nucifera |
| | Time of collection | | | | | |
| | Mid-Nov. | Late May | Late June | Mid-May | Early Apr. | Late Nov. |
| Purified polyprenyl acetate Yield (g) | 0.2 | 0.9 | 5.3 | 2.4 | 2.0 | 1.8 |
| Purity (%) | >90 | >90 | >90 | >90 | >90 | >90 |

Example 15

Leaves of Sciadopitys verticillata (10 kg; undried weight) as collected in Wakayama Prefecture in mid-April were air-dried at about 50° C for 24 hours and then immersed in 80 liters of chloroform at room temperature (about 15° C) for a week for extraction. The chloroform was then distilled off from the extract, 5 liters of hexane was added to the concentrate thus obtained, the insoluble matter was filtered off, and the filtrate was concentrated and chromatographed on a silica gel column using chloroform as the developing solvent to give about 23 gof an oil as a fraction exhibiting RF values of 0.51 and 0.20 in thin layer chromatography performed under the same conditions as in Example 1. To the above oil was added about 400 ml of acetone for dissolving acetone-soluble components, the resulting mixture was filtered, and the filtrate was concentrated. The residual liquid was heated with 400 ml of methanol, 40 ml of water and 30 g of sodium hydroxide at 65° C for 2 hours. Then the methanol was distilled off, 400 ml of diethyl ether was added to the residue for extraction, the ether layer was washed five times with about 100 ml of saturated aqueous sodium chloride and then dried over anhydrous sodium sulfate, and the solvent was distilled off to give 18.2 g of a liquid. This liquid was chromatographed using about 1 kg of silica gel and a n-hexane-diisopropyl ether (90 : 10 by volume) mixture to give 15.3 g of a liquid as a fraction showing an Rf value of 0.20 in the thin layer chromatography mentioned above. This liquid was a polyprenol fraction having a purity of not less than 90%. The molecular weight distribution as measured therefor by the same method as used in Example 1 was as follows:

| Peak No. | Value of n (Number of cis-isoprene units) | Area proportion (%) |
|---|---|---|
| 1 | 10 | 0.6 |
| 2 | 11 | 1.2 |
| 3 | 12 | 5.0 |
| 4 | 13 | 18.7 |
| 5 | 14 | 29.9 |
| 6 | 15 | 22.9 |
| 7 | 16 | 7.3 |
| 8 | 17 | 2.9 |
| 9 | 18 | 3.5 |
| 10 | 19 | 2.4 |
| 11 | 20 | 2.2 |
| 12 | 21 | 2.3 |
| 13 | 22 | 1.0 |

Each component was isolated from the above oily product by the same high performance liquid chromatography as conducted in Example 1 and identified as a polyprenol of general formula (I) wherein A is a hydroxyl group by mass spectrometry, infrared spectroscopy, $^{1}H$-NMR spectroscopy and $^{13}C$-NMR spectroscopy.

For each of the polyprenols thus isolated and having n values of 11 to 22, respectively, the FD-MASS m/e value and the $\delta$ values (ppm) for the characteristic signals found in $^{1}H$-NMR and $^{13}C$-NMR spectroscopy were in good agreement with those described in Table 1 and Table 2. The FD-MASS m/e value for the polyprenol having the n value of 10 was 902. The $\delta$ values (ppm) of the characteristic signals for this polyprenol as found in $^{1}H$-NMR and $^{13}C$-NMR spectroscopy were in substantial agreement with those for the polyprenols in which n = 11—22.

Example 16

Leaves of Cephalotaxus harringtonia subsp. nana (5 kg; undried weight) as collected in Okayama Prefecture late April were dried with hot air, finely divided in a mixer and extracted with 40 liters of a petroleum ether-acetone (4 : 1 by volume) mixture at room temperature (about 20° C) for 5 days. The extract was washed with water and dried over anhydrous sodium sulfate, and then the solvent was distilled off to give about 100 g of a residue. To this was added one liter of n-hexane for dissolving n-hexane-soluble components, the mixture was filtered, and the filtrate was concentrated and chromatographed using a silica gel column and a n-hexane-diethyl ether (95 : 5 by volume) mixture to give about 3.2 g of a liquid as a fraction showing Rf values of 0.50 and 0.19 in the same thin layer chromatography as used in Example 1. About 50 ml of acetone was added to this liquid for dissolving acetone-soluble components, the mixture was filtered, the filtrate was concentrated, and the liquid thus obtained was heated with 100 ml of methanol, 10 ml of water and 5 g of potassium hydroxide at 65° C for 2 hours. The methanol was then distilled off, 200 ml of hexane was added to the residue for extraction, the hexane layer was washed five times with about 50 ml of saturated aqueous sodium chloride, and the solvent was distilled off to give 2.7 g of a liquid. This liquid was chromatographed using about 100 g of silica gel and chloroform to give 2.2 g of a liquid as a fraction showing an Rf value of 0.20 in the above thin layer chromatography. The oil was a polyprenol fraction having a purity of not less than 90%. The molecular weight distribution as measured therefor by the same method as used in Example 1 was as follows.

| Peak No. | Value of n (Number of cis-isoprene units) | Area proportion (%) |
|---|---|---|
| 1 | 12 | 1.7 |
| 2 | 13 | 9.2 |
| 3 | 14 | 27.8 |
| 4 | 15 | 35.0 |
| 5 | 16 | 14.3 |
| 6 | 17 | 4.8 |
| 7 | 18 | 2.5 |
| 8 | 19 | 2.6 |
| 9 | 20 | 2.1 |

Each component was isolated from this liquid by the same high performance liquid chromatography as in Example 1. Mass spectrometry, infrared spectroscopy, $^1H$-NMR spectroscopy and $^{13}C$-NMR spectroscopy gave analytical data which were in substantial agreement with those for the corresponding component (equal in value of n) obtained in Example 1.

Example 17

The procedure of Example 16 was followed except that the saponification with alkali hydroxide was omitted. The filtrate concentrate was directly subjected to column chromatography using about 100 g of silica gel and chloroform to give 2.1 g of a liquid as a fraction exhibiting an Rf value of 0.51 in the above thin layer chromatography. The liquid was a polyprenyl acetate fraction having a purity of not less than 90%. The molecular weight distribution as measured therefor was as shown below. This molecular weight distribution was determined based on the area proportions of individual peaks in the chromatogram obtained with the high performance liquid chromatographic column used in Example 1 and an acetone-methanol (70 : 30 by volume) mixture eluent.

| Peak No. | Value of n (Number of cis-isoprene units) | Area proportion (%) |
|---|---|---|
| 1 | 12 | 1.5 |
| 2 | 13 | 9.3 |
| 3 | 14 | 27.9 |
| 4 | 15 | 35.3 |
| 5 | 16 | 14.4 |
| 6 | 17 | 4.9 |
| 7 | 18 | 2.3 |
| 8 | 19 | 2.3 |
| 9 | 20 | 2.1 |

Each component was isolated from the above liquid (polyprenyl acetate content: not less than 90%) by the same high performance liquid chromatography as in Example 1 and identified as a polyprenyl acetate of general formula (I) wherein A is an acetoxy group by mass spectrometry, infrared spectroscopy, $^{1}H$-NMR spectroscopy and $^{13}C$-NMR spectroscopy. The results of the FD-MASS analysis for the respective components are shown in Table 5.

Table 5

| Value of n (Number of cis-isoprene units) | FD-MASS (m/e) Found | Calcd. |
|---|---|---|
| 12 | 1080 | 1080 |
| 13 | 1148 | 1148 |
| 14 | 1216 | 1216 |
| 15 | 1284 | 1284 |
| 16 | 1352 | 1352 |
| 17 | 1420 | 1420 |
| 18 | 1488 | 1488 |
| 19 | 1556 | 1556 |
| 20 | 1624 | 1624 |

Each component was hydrolyzed by the same procedure as used in Example 16, and the polyprenol obtained gave quite the same $^{1}H$-NMR spectrum, 13C-NMR spectrum and infrared absorption spectrum as those for the corresponding polyprenol (equal in value n) obtained in Example 16.

Example 18

Leaves of Araucaria brasiliana (5 kg; undried weight) as collected in Okayama Prefecture early April were dried with hot air, then finely divided, and extracted with 40 liters of ethyl acetate at room temperature (about 15°C) for 5 days. The extract was concentrated, and about 500 ml of hexane was added to the concentrate for dissolving soluble components. After filtration, the filtrate was concentrated to give a dark brown liquid. This liquid was heated with 500 ml of methanol, 50 ml of water and 25 g of sodium hydroxide at 65°C for 2 hours. The mixture was cooled to room temperature and extracted with 500 ml of hexane. The hexane layer was washed with about 150 ml of saturated aqueous sodium chloride and dried over anhydrous magnesium sulfate, and then the solvent was distilled off to give about 17 g of a liquid. This liquid was purified by column chromatography using about 1 kg of silica gel with methylene chloride as the developing solvent to give 7.2 g of a liquid. This liquid was a polyprenol fraction having a purity of not less than 90%. The molecular weight distribution therefore as measured by the same method as in Example 1 was as follows:

| Peak No. | Value of n (Number of cis-isoprene units) | Area proportion (%) |
|---|---|---|
| 1 | 10 | 0.6 |
| 2 | 11 | 1.5 |
| 3 | 12 | 4.8 |
| 4 | 13 | 12.0 |
| 5 | 14 | 12.2 |
| 6 | 15 | 6.5 |
| 7 | 16 | 5.0 |
| 8 | 17 | 4.5 |
| 9 | 18 | 7.5 |
| 10 | 19 | 14.6 |
| 11 | 20 | 19.0 |
| 12 | 21 | 9.5 |
| 13 | 22 | 2.3 |

Each component was isolated from the above liquid by the same high performance liquid chromatography as in Example 1. The mass spectrometry, infrared spectroscopy, $^1$H-NMR spectroscopy and $^{13}$C-NMR spectroscopy data therefor were in substantial agreement with those for the corresponding component (equal in value of n) obtained in Example 1.

Example 19

Leaves of Podocarpus macrophylla (5 kg; undried weight) as collected in Okayama Prefecture early April were treated by the same procedure as in Example 18 to give 5.7 g of a liquid. This was a polyprenol fraction having a purity of not less than 90%. The molecular weight distribution as measured therefor by the same method as in Example 1 was as follows:

| Peak No. | Value of n (Number of cis-isoprene units) | Area proportion (%) |
|---|---|---|
| 1 | 10 | 0.5 |
| 2 | 11 | 1.6 |
| 3 | 12 | 5.0 |
| 4 | 13 | 11.4 |
| 5 | 14 | 10.5 |
| 6 | 15 | 6.2 |
| 7 | 16 | 2.0 |
| 8 | 17 | 1.0 |
| 9 | 18 | 2.3 |
| 10 | 19 | 7.1 |
| 11 | 20 | 19.9 |
| 12 | 21 | 23.5 |
| 13 | 22 | 9.1 |

Each component was isolated from the above liquid by the same high performance liquid chromatography as in Example 1. The mass spectrometry, infrared spectroscopy, $^1$H-NMR spectroscopy and $^{13}$C-NMR spectroscopy data therefor were in substantial agreement with those for the corresponding component (equal in value of n) obtained in Example 1.

Examples 20—47

Leaves of a starting plant were air-dried at about 40° C for 12 hours and divided into 100-g portions. Each portion was immersed in one liter of a solvent (specified in Table 6) at room temperature for 7 days for exraction. The extractant solvent was distilled off from the extract, the residue was dissolved in 200 ml of hexane, the solution was washed three times with about 100 ml of a methanol-water (9 : 1 by volume) mixture and then dried over anhydrous magnesium sulfate, and the solvent was distilled off to give an oil. The oil was heated with 20 ml of methanol and 0.5 g of potassium hydroxide at 65° C for 2 hours. The methanol was then distilled off, and 50 ml of diethyl ether was added to the residue for extraction. The ether layer was washed three times with about 20 ml of saturated aqueous sodium chloride and dried over anhydrous magnesium sulfate, and then the solvent was distilled off to give an oil. This oil was chromatographed using 100 g of silica gel and a n-hexane-ethyl acetate (9 : 1 by volume) mixture to give a fraction (polyprenol mixture) showing an Rf value of 0.19 in the same thin layer chromatography as in Example 1. The weight of this fraction was regarded as the polyprenol yield, which is shown in Table 6. The polyprenol mixtures obtained in this manner had substantially the same compositions as the corresponding polyprenol mixtures obtained in Examples 2—7 from the same starting plants had.

Table 6

| Example No. | Raw material plant species | Extracting solvent*) | Polyprenol yield (g) |
|---|---|---|---|
| 20 | Cryptomeria japonica | Benzene | 0.79 |
| 21 | Cryptomeria japonica | Ethyl acetate | 0.81 |
| 22 | Cryptomeria japonica | Isopropyl alcohol | 0.54 |
| 23 | Cryptomeria japonica | Acetone-chloroform = 50 : 50 | 0.65 |
| 24 | Metasequoia glyptostroboides | Benzene | 0.18 |
| 25 | Metasequoia glyptostroboides | Ethyl acetate | 0.21 |
| 26 | Metasequoia glyptostroboides | Isopropyl alcohol | 0.14 |
| 27 | Metasequoia glyptostroboides | Acetone-chloroform = 50 : 50 | 0.17 |
| 28 | Chamaecyparis obtusa | Benzene | 0.77 |
| 29 | Chamaecyparis obtusa | Ethyl acetate | 0.73 |
| 30 | Chamaecyparis obtusa | Isopropyl alcohol | 0.55 |
| 31 | Chamaecyparis obtusa | Acetone-chloroform = 50 : 50 | 0.58 |
| 32 | Juniperus chinensis | Benzene | 0.03 |
| 33 | Juniperus chinensis | Ethyl acetate | 0.04 |
| 34 | Juniperus chinensis | Isopropyl alcohol | 0.03 |
| 35 | Juniperus chinensis | Acetone-chloroform = 50 : 50 | 0.04 |
| 36 | Juniperus rigida | Benzene | 0.42 |
| 37 | Juniperus rigida | Ethyl acetate | 0.47 |
| 38 | Juniperus rigida | Isopropyl alcohol | 0.31 |
| 39 | Juniperus rigida | Acetone-chloroform = 50 : 50 | 0.39 |
| 40 | Taxus cuspidata | Benzene | 0.29 |
| 41 | Taxus cuspidata | Ethyl acetate | 0.36 |
| 42 | Taxus cuspidata | Isopropyl alcohol | 0.22 |
| 43 | Taxus cuspidata | Acetone-chloroform = 50 : 50 | 0.34 |
| 44 | Torreya nucifera | Benzene | 0.32 |
| 45 | Torreya nucifera | Ethyl acetate | 0.33 |
| 46 | Torreya nucifera | Isopropyl alcohol | 0.17 |
| 47 | Torreya nucifera | Acetone-chloroform = 50 : 50 | 0.29 |

*) The numerical ratio value for each mixed solvent indicates the ratio by volume between the solvents used.

Examples 48—68

Leaves of Cephalotaxus harringtonia subsp. nana as collected in Okayama Prefecture late April were air-dried at about 60°C for 65 hours and divided into 100-g portions. Each portion of leaves was extracted with one liter of a solvent (specified in Table 7) at room temperature (about 20° C) for 7 days. The extraction solvent was distilled off from the extract, and the residue was weighed. The residue weiht is shown in the »Total extract weight« column in Table 7. This extract (residue) was dissolved in 200 ml of hexane, the solution was washed three times with about 100 ml of a methanol-water (9 : 1 by volume) mixture and dried over anhydrous magnesium sulfate, and the solvent was then distilled off to give an oil. The oil was heated with 50 ml of methanol and 1 g of potassium hydroxide at 65°C for 2 hours. Then, the methanol was distilled off, 100 ml of diethyl ether was added to the residue for extraction, the ether layer was washed three times with about 50 ml of saturated aqueous sodium chloride and dried over anhydrous magnesium sulfate, and the solvent was distilled off to give an oil. This oil was chromatographed using 100 g of silica and a n-hexane-ethyl acetate (9 : 1 by volume) mixture to give a fraction (polyprenol mixture) giving an Rf value of 0.19 in the same thin layer chromatography as in Example 1. The weight of this fraction is shown in the »Polyprenol content« column in Table 7. The composition of the polyprenol mixture thus obtained was in substantial agreement with that of the polyprenol mixture obtained in Example 16 without depending on the kind of the solvent used.

Table 7

| Example No. | Solvent | Total extract weight (g) | Polyprenol content (g) |
|---|---|---|---|
| 48 | n-Hexane | 4.28 | 0.52 |
| 49 | Petroleum ether | 4.36 | 0.52 |
| 50 | Benzene | 6.09 | 0.51 |
| 51 | Chloroform | 7.28 | 0.49 |
| 52 | Carbon tetrachloride | 5.42 | 0.41 |
| 53 | Diethyl ether | 9.31 | 0.53 |
| 54 | Tetrahydrofuran | 11.21 | 0.41 |
| 55 | Methanol | 16.31 | 0.11 |
| 56 | Ethanol | 12.11 | 0.20 |
| 57 | Isopropyl alcohol | 6.20 | 0.25 |
| 58 | Acetone | 7.80 | 0.51 |
| 59 | Ethyl acetate | 6.39 | 0.50 |
| 60 | Acetone hexane (20 : 80 by volume) | 5.88 | 0.50 |
| 61 | Acetone-hexane (50 : 50 by volume) | 7.92 | 0.51 |
| 62 | Acetone-chloroform (20 : 80 by volume) | 7.65 | 0.49 |
| 63 | Acetone-chloroform (50 : 50 by volume) | 7.31 | 0.51 |
| 64 | Methanol-chloroform (20 : 80 by volume) | 15.16 | 0.50 |
| 65 | Methanol-chloroform (50 : 50 by volume) | 17.20 | 0.42 |
| 66 | Diethyl ether-hexane (20 : 80 by volume) | 6.15 | 0.44 |
| 67 | Methanol-acetone (20 : 80 by volume) | 10.32 | 0.46 |
| 68 | Methanol-acetone (50 : 50 by volume) | 12.19 | 0.29 |

## Example 69

To a solution of 1.24 g of a polyprenol of general formula (I) wherein n = 15 and A = OH as obtained from the leaves fo Cryptomeria japonica by the procedure of Example 1 and 1.0 g of pyridine in dried diethyl ether, there was added dropwise 1.2 g of acetic anhydride at room temperature. After completion of the dropping, the mixture was stirred at room temperature overnight. The reaction mixture was washed with saturated aqueous sodium chloride and dried over anhydrous magnesium sulfate, and the diethyl ether was distilled off to give a light-yellow viscous oil. This oil was purified by silica gel column chromatography (developing solvent: hexane-ethyl acetate) to give 1.04 g of a pale yellow liquid. IR analysis of this liquid revealed that the absorption at about 3,300 $cm^{-1}$ due to the hydroxyl group of the starting polyprenol had disappeared and absorptions assignable to $-OCOCH_3$ had appeared at 1,745-$cm^{-1}$ and 1,255 $cm^{-1}$, NMR analysis showed that the signal assignable to $-C\underline{H}_2OH$ of the starting polyprenol (doublet, $\delta$=4.08) had disappeared and a new signal (doublet, $\delta$=4.55) assignable to $-C\underline{H}_2OCOCH_3$ was observed. The signal assignable to $-CH_2OCOC\underline{H}_3$ was observed overlapping with the signal ($\delta$=2.04) assignable to

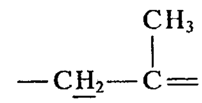

FD-MASS analysis gave m/e = 1,284. Based on the above data, the above liquid was identified as a polyprenyl acetate of general formula (I) wherein n = 15 and A = $OCOCH_3$.

The above procedure was followed using 1.45 g of a polyprenol of general formula (I) wherein n = 18 and A = OH as obtained from the leaves of Chamaecyparis obtusa in the same manner as in Example 3, to give 1.12 g of a pale yellow liquid. This liquid was identified as a polyprenyl acetate of general formula (I) wherein n = 18 and A = $OCHCH_3$. FD-MASS analysis gave m/e = 1,488.

Polyprenyl acetates of general formula (I) wherein n is other than the above values and polyprenyl acetate mixtures having various compositions with n being distributed in the range of 11—21 can also be synthesized by the above procedure.

## Example 70

A solution of 1.24 g of a polyprenol of general formula (I) wherein n = 15 and A = OH as obtained from the leaves of Chamaecyparis obtusa in the same manner as in Example 3, 0.5 g of methyl oleate and 0.01 g of sodium hydride in 50 ml of toluene was heated in a nitrogen atmosphere at 110° C for 48 hours. The reaction mixture was cooled to room temperature, washed with saturated aqueous sodium chloride and dried over anhydrous magnesium sulfate, and then the solvent was distilled off to give a yellow liquid. This liquid was purified by silica gel column chromatography (developing solvent: hexane-ethyl acetate) to give 0.45 g of a colorless, viscous liquid. IR analysis of the liquid showed that the absorption at about 3,300 $cm^{-1}$ due to the OH group of the starting polyprenol had disappeared. FD-MASS analysis gave m/e = 1,506. Based on the above, this liquid was identified as a polyprenyl oleate of general formula (I) wherein n = 15 and A = $OCOC_{17}H_{33}$. Polyprenyl oleates having n values other than 15 and polyprenyl oleates mixtures having various compositions with n being distributed in the range of 11—21 can also be synthesized by the above procedure.

## Example 71

To a mixture of 1.17 g of a polyprenol of general formula (I) wherein n = 14 and A = OH as obtained from the leaves of Juniperus rigida in the same manner as in Example 5 and 10 ml of pyridine, there was added 0.28 g of benzoyl chloride at room temperature, and the whole mixture was stirred at room temperature overnight. The reaction mixture was then poured into about 150 ml of water and extracted with diethyl ether. The ether layer obtained was washed in sequence with saturated aqueous sodium chloride, diluted hydrochloric acid, saturated aqueous sodium hydrogen carbonate and saturated aqueous sodium chloride, and dried over anhydrous magnesium sulfate, and then the diethyl ether was distilled off to give a yellow liquid. This liquid was purified by silica gel column chromatography (developing solvent: hexane-ethyl acetate) to give 0.87 g of a pale yellow liquid. IR analysis of this liquid revealed that the absorption due to the OH group of the starting polyprenol had disappeared and new absorptions due to the ester bonding had appeared at 1,715 $cm^{-1}$ and 1,270 $cm^{-1}$. FD-MASS analysis gave m/e = 1,278. Based on the above, the above liquid was identified as a polyprenyl benzoate of general formula (I) wherein n = 14 and A = $OCOC_6H_5$. Polyprenyl benzoates having n values other than 14 as well as polyprenyl benzoate mixtures having various compositions with n being distributed in the range of 11—20 can also be synthesized by the above procedure.

Example 72

To a solution of 1.17 g of a polyprenol of general formula (I) wherein n = 14 and A = OH as obtained from the leaves of Sciadopitys verticillata in the same manner as in Example 15 and 1.0 g of pyridine in dried diethyl ether, there was added dropwise 1.2 g of acetic anhydride at room temperature. After completion of the dropping, the mixture was stirred at room temperature overnight. The reaction mixture was washed with saturated aqueous sodium chloride and dried over anhydrous magnesium sulfate, and then the diethyl ether was distilled off to give a light-yellow viscous liquid. This was Purified by silica gel column chromatography (developing solvent: hexane-ethyl acetate) to give 1.01 g of a pale yellow liquid. IR analysis of this liquid showed that the absorption due to the OH group of the starting polyprenol (at about 3,300 $cm^{-1}$) had disappeared and new absorptions had appeared at 1,745 $cm^{-1}$ and 1,255 $cm^{-1}$. In NMR analysis, the signal (doublet, $\delta$=4.08) assignable to $-\underline{CH_2}OH$ of the starting polyprenol was no more observed but a new signal (doublet, $\delta$=4.55) assignable to $-\underline{CH_2}OCOCH_3$ was observed. The signal assignable to $-CH_2OCO\underline{CH_3}$ was observed overlapping with the signal ($\delta$=2.04) assignable to

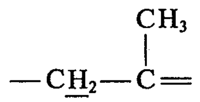

Based on the above, the above liquid was identified as a polyprenyl acetate of general formula (I) wherein n = 14 and A = $OCOCH_3$. Polyprenyl acetates having n values other than 14 and polyprenyl acetate mixtures having various compositions with n being distributed in the range of 10—22 can also be synthesized by the above procedure.

Example 73

A solution of 1.17 g of a polyprenyl of general formula (I) wherein n = 14 and A = OH as obtained from the leaves of Sciadopitys verticillata in the same manner as in Example 15, 0.5 g of methyl oleate and 0.01 g of sodium hydride in 50 ml of toluene was heated in a nitrogen atmosphere at 110° C for 24 hours. The reaction mixture was cooled to room temperature, washed with saturated aqueous sodium chloride and dried over anhydrous magnesium sulfate, and then the solvent was distilled off to give a yellow liquid. This liquid was purified by silica gel column chromatography (developing solvent: hexane-ethyl acetate) to give 0.44 g of a colorless viscous liquid. IR analysis of this liquid revealed that the absorption at about 3,300 $cm^{-1}$ due to the OH group of the starting polyprenol had disappeared. FD-MASS analysis gave m/e = 1,438. Based on the above, said liquid was identified as a polyprenyl acetate of general formula (I) wherein n = 14 and A = $OCOC_{17}H_{33}$. Polyprenyl oleates having n values other than 14 and polyprenyl oleate mixtures having various compositions with n being distributed in the range of 10—22 can also be synthesized by the above procedure.

Example 74

To a mixture of 1.31 g of a polyprenol of general formula (I) wherein n = 16 and A = OH as obtained from the leaves of Cephalotaxus harringtonia subsp. nana by the procedure of Example 16 and 10 ml of pyridine, there was added 0.28 g of benzoyl chloride at room temperature. The whole mixture as stirred at room temperature overnight. The reaction mixture as then poured into about 150 ml of water and extracted with diethyl ether. The ether layer obtained was washed with saturated aqueous sodium chloride, diluted hydrochloric acid, saturated aqueous sodium hydrogen carbonate and saturated aqueous sodium chloride in that order and dried over anhydrous magnesium sulfate. The diethyl ether was then distilled off to give a yellow liquid. This was purified by silica gel column chromatography (developing solvent: hexane-ethyl acetate) to give 0.79 g of a pale yellow liquid. IR analysis of this liquid showed that the absorption due to the OH group of the starting polyprenol had disappeared and new absorptions due to the ester bonding had appeared at 1,715 $cm^{-1}$ and 1,270 $cm^{-1}$. FD-MASS analysis gave m/e = 1,414. Based on the above, the above liquid was identified as a polyprenyl benzoate of general formula (I) wherein n = 16 and A = $OCOC_6H_5$. Polyprenyl benzoates of general formula (I) having n values other than 16 and polyprenyl benzoate mixtures having various compositions with n being distributed in the range of 12—20 can also be synthesized by the above procedure.

Reference Example

A three-necked flask purged with argon was charged with 0.316 g (13 millimoles) of magnesium flakes, 0.5 ml of anhydrous tetrahydrofuran and 0.08 ml of 1,2-dibromoethane, and the contents were

heated by a dryer until vigorous bubbling occurred. A solution of 2.51 g (10 millimoles) of 2-[4-bromo-3-methylbutoxy]-tetrahydro-2H-pyrane in 3.0 ml of anhydrous tetrahydrofuran was added dropwise to the above activated magnesium at such a speed that the solvent was just boiled. After completion of the dropping, the mixture was stirred at 70° C for 15 minutes. Then, 60 ml of anhydrous tetrahydrofuran was added to form a Grignard solution.

Another three-necked flask purged with argon was charged with a solution of 6.42 g (5 millimoles) of the polyprenyl acetate of general formula (I) in which n is 15 and A is $-OCOCH_3$ in 15 ml of anhydrous tetrahydrofuran, and 2.0 ml of a 0.1M anhydrous tetrahydrofuran solution of $Li_2CuCl_4$. Then, the Grignard solution prepared as above was added dropwise at 0° C over 1 hour, and then the mixture was stirred at 0° C for 2 hours. Thereafter, a saturated aqueous solution of ammonium chloride was added to the reaction mixture to effect hydrolysis, and the product was extracted with diethyl ether. The diethyl ether layer was washed with a saturated aqueous solution of sodium chloride, and dried over anhydrous magnesium sulfate. The solvent was then removed by means of a rotary evaporator to give 7.95 g of a pale yellow liquid. In silica gel thin-layer chromatography (hexane/ethyl acetate = 97/3 as a developing solvent), this liquid was found to give a main spot at Rf = 0.35. FD-MASS analysis of the pale yellow liquid did not give the m/e = 1284 value indicating the presence of the starting polyprenyl acetate, but gave m/e = 1396 as a main peak, which showed the presence of the desired compound of general formula (III) in which Z is a tetrahydro-2H-pyranyl group.

The pale yellow liquid was then dissolved in 40 ml of hexane, and 0.13 g (0.5 millimole) of pyridinium p-toluenesulfonate and 20 ml of ethanol were added. The solution was heated at 55° C for 3 hours with stirring. The reaction mixture was cooled, and then neutralized with 0.21 g of sodium carbonate. The solvent was distilled off by a rotary evaporator. The resulting concentrate was dissolved in diethyl ether, washed with a saturated aqueous solution of sodium hydrogen carbonate and then with a saturated aqueous solution of sodium chloride, and then dried over anhydrous magnesium sulfate. The solvent was removed by means of a rotary evaporator. The remaining oily substance was heated at 150° C and 0.5 torr for 30 minutes to remove low-boiling components. The remaining oil was chromatographed on a silica gel column using hexane/ethyl acetate (9/1) as an eluent to vive 5.64 g of a colorless transparent liquid. In silica gel thin layer chromatography (hexane/ethyl acetate = 9/1 as a developing solvent), this liquid showed a single spot at Rf = 0.19. Based on the analytical data shown below, this liquid was identified as the compound of general formula (A) in which j is 15.

FD-MASS:
m/e = 1312 (calculated 1312)
IR ($cm^{-1}$):
830, 1060, 1376, 1440, 2850, 2920, 3320.
$^{13}C$-NMR (ppm/intensity):
135.365/430, 135.229/3567, 135.005/349, 134.937/290, 131.210/213, 125.071/5242, 124.993/499, 124.448/505, 124.282/463, 124.214/445, 61.241/551, 40.029/541, 39.757/683, 37.548/582, 32.245/5500, 32.021/456, 29.316/528, 26.825/492, 26.699/548, 26.436/5166, 25.677/542, 25.308/567, 23.430/6330, 19.557/548, 17.679/353, 16.006/640.
$^1H$-NMR (ppm, signal form, proton ratio):
5.10 (b, 18H), 3.66 (m, 2H), 2.03 (b, 70H), 1.68 (s, 48H), 1.60 (s, 9H), 1.80—1.10 (m, 5H), 0.91 (d, 3H).

## Claims

1. A method of preparing a polyprenyl compound or a mixture of polyprenyl compounds, represented by the general formula

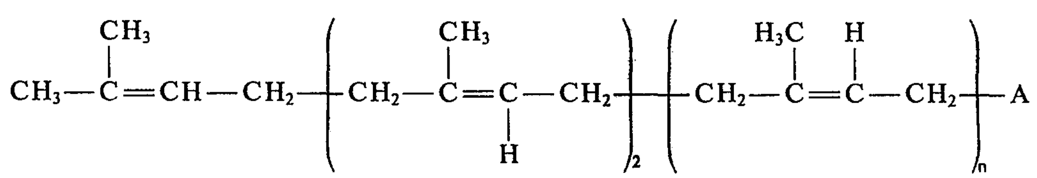
(I)

wherein A is a hydroxyl or acyloxy group,

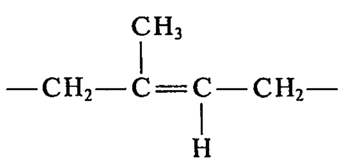

represents a trans-isoprene unit,

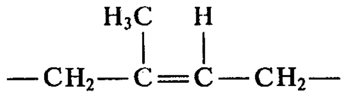

represents a cis-isoprene unit and n is an integer of 10 to 22, which comprises extracting the leaves of a plant belonging to the family Cupressaceae, Taxodiaceae, Sciadopitydaceae, Podocarpaceae, Cephalotaxaceae, Araucariaceae or Taxaceae with an organic solvent and isolating a polyprenyl compound or a mixture of polyprenyl compounds from the extract, if necessary following or followed by subjecting the extract or the isolated polyprenyl compound or mixture to one or more reactions selected from among hydrolysis, esterification and transesterification.

2. The method of Claim 1, wherein the organic solvent is selected from the group consisting of hydrocarbons, halogenated hydrocarbons, ethers, esters and ketones.

3. The method of Claim 1, wherein the extract obtained by extracting the leaves of said plant with an organic solvent is, if necessary after hydrolysis treatment, subjected to a separation step comprising chromatography, fractional dissolution, fractional refrigerating precipitation or molecular distillation or a combination of two or more of these, whereby a fraction giving an Rf value of from 0.18 to 0.25 in thin layer chromatography and essentially consisting of polyprenyl compounds of general formula (I) wherein A is a hydroxyl group and/or a polyprenyl fraction giving an Rf value of from 0.50 to 0.55 in thin layer chromatography and essentially consisting of compounds of general formula (I) wherein A is an acetoxy group are/is recovered, said thin layer chromatography being carried out on a Merck plate for thin layer chromatography (coated with silica gel $60F_{254}$; layer thickness 0.25 mm) with a mixture of n-hexane and ethyl acetate in a volume ratio of 9 : 1 as a developing solvent (developed 10 cm) under the conditions such that solanesyl acetate as a standard substance gives an Rf value of from 0.40 to 0.45.

4. The method of Claim 1, wherein the plant is a member selected from the group consisting of plants belonging to the genera Chamaecyparis, Juniperus, Cryptomeria, Metasequoia, Sciadopitys, Podocarpus, Cephalotaxus, Agathis, Araucaria, Taxus and Torreya.

## Patentansprüche

1. Verfahren zur Herstellung einer Polyprenylverbindung oder einem Gemisch von Polyprenylverbindungen der allgemeinen Formel

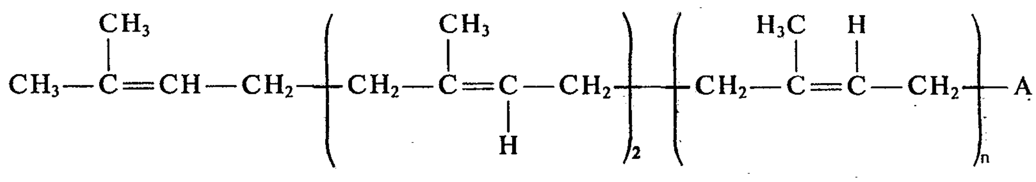

(I)

in der A eine Hydroxylgruppe oder einen Acyloxyrest bedeutet,

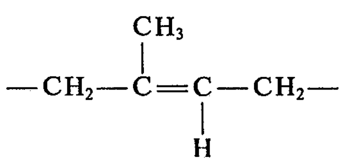

eine trans-Isopreneinheit bedeutet,

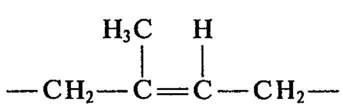

eine cis-Isopreneinheit bedeutet und n eine ganze Zahl von 10 bis 22 ist, dadurch gekennzeichnet, daß man die Blätter einer Pflanze der Familie Cupressaceae, Taxodiaceae, Sciadopitydaceae, Podocarpaceae, Cephalotaxaceae, Araucariaceae oder Taxaceae mit einem organischen Lösungsmittel extrahiert und eine Polyprenylverbindung oder ein Gemisch von Polyprenylverbindungen aus dem Extrakt isoliert und gegebenenfalls vorher oder anschließend den Extrakt oder die isolierte Polyprenylverbindung oder deren Gemische einer oder mehrerer Reaktionen unterwirft, die ausgewählt sind aus einer Hydrolyse-, Veresterungs- und Umesterungsreaktion.

2. Verfahren nach Anspruch 1, wobei das organische Lösungsmittel ausgewählt ist aus der Gruppe bestehend aus Kohlenwasserstoffen, halogenierten Kohlenwasserstoffen, Äthern, Estern und Ketonen.

3. Verfahren nach Anspruch 1, wobei der durch Extraktion der Blätter besagter Pflanzen mit einem organischen Lösungsmittel erhaltene Extrakt, falls erforderlich, nach der Hydrolysebehandlung einer Trennungsstufe unterworfen wird, wie Chromatographie, fraktionierte Auflösung, fraktionierte Kältefällung oder Molekulardestillation oder einer Kombination von zwei oder mehreren dieser Trennungsverfahren, wobei eine Fraktion mit einem $R_f$-Wert von 0,18 bis 0,25 bei der Dünnschichtchromatographie gewonnen wird, die hauptsächlich aus Polyprenylverbindungen der allgemeinen Formel I besteht, in der A eine Hydroxylgruppe ist, und/oder eine Polyprenylfraktion mit einem $R_f$-Wert von 0,50 bis 0,55 bei der Dünnschichtchromatographie erhalten wird, die hauptsächlich aus Verbindungen der allgemeinen Formel I besteht, in der A eine Acetoxygruppe ist, und diese Dünnschichtchromatographie an einer Merck-Platte für Dünnschichtchromatographie (beschichtet mit Silikagel $60F_{254}$; Schichtdicke 0,25 mm) mit einem Gemisch von n-Hexan und Äthylacetat in einem Volumenverhältnis von 9 : 1 als Entwicklungslösungsmittel (10 cm entwickelt) unter solchen Bedingungen durchgeführt wird, daß Solanesylacetat als Standardsubstanz einen $R_f$-Wert von 0,40 bis 0,45 aufweist.

4. Verfahren nach Anspruch 1, wobei die Pflanze ausgewählt ist aus der Gruppe bestehend aus Pflanzen, die der Gattung Chamaecyparis, Juniperus, Cryptomeria, Metasequoia, Sciadopitys, Podocarpus, Cephalotaxus, Agathis, Araucaria, Taxus und Torreya angehören.

**Revendications**

1. Un procédé de préparation d'un composé polyprénylique ou d'un mélange de composés polyprényliques, représentés par la formule générale:

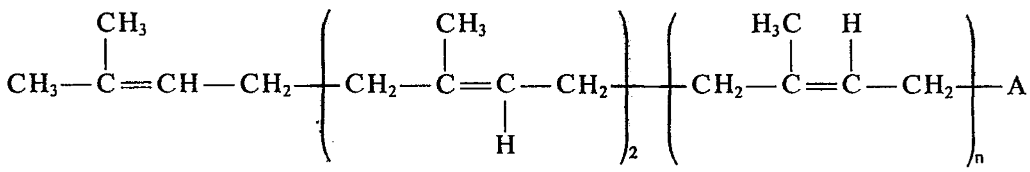

(I)

dans laquelle A est un groupe hydroxyle ou acyloxy,

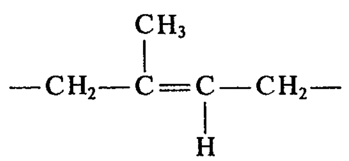

représente un motif de trans-isoprène,

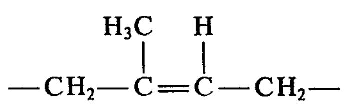

représente un motif de cis-isoprène, et n est un entier de 10 à 22, qui comprend l'extraction des feuilles d'une plante appartenant à la famille des Cupressaceae, Taxodiaceae, Sciadopitydaceae, Podocarpaceae, Cephalotaxaceae, Araucariaceae ou Taxaceae avec un solvant organique, et l'isolement d'un composé polyprénylique ou d'un mélange de composés polyprényliques de l'extrait, au besoin avant ou après avoir soumis l'extrait ou le composé polyprénylique isolé ou le mélange à une ou plusieurs réactions choisies parmi l'hydrolyse, l'estérification et la transestérification.

2. Le procédé de la revendication 1, dans lequel le solvant organique est choisi dans le groupe constitué par les hydrocarbures, les hydrocarbures halogénés, les éthers, les esters et les cétones.

3. Le procédé de la revendication 1, dans lequel l'extrait obtenu par extraction des feuilles de ladite plante avec un solvant organique est, au besoin, après traitement d'hydrolyse, soumis à un stade de séparation comprenant une chromatographie, une dissolution fractionnée, une précipitation par réfrigération fractionnée ou une distillation moléculaire ou une combinaison de deux ou plusieurs de ces stades, pour qu'une fraction présentant un Rf de 0,18 à 0,25 en chromatographie en couche mince et constituée essentiellement de composés polyprényliques de formule générale (I), dans laquelle A est un groupe hydroxyle, et/ou une fraction polyprénylique présentant un Rf de 0,50 à 0,55 en chromatographie en couche mince et constituée essentiellement de composés de formule générale (I), dans laquelle A est un groupe acétoxy, soient/soit récupérée(s), ladite chromatographie en couche mince étant effectuée sur une plaque Merck pour chromatographie en couche mince (revêtue de gel de silice $60F_{254}$; épaisseur de la couche 0,25 mm) avec un mélange de n-hexane et d'acétate d'éthyle dans un

rapport en volume de 9/1 comme solvant de développement (développement sur 10 cm) dans des conditions telles que l'acétate de solanésyle, servant de substance standard, présente un Rf de 0,40 à 0,45.

4. Le procédé de la revendication 1, dans lequel la plante est choisie dans le groupe constitué par les plantes appartenant aux genres Chamaecyparis, Juniperus, Cryptomeria, Metasequoia, Sciadopitys, Podocarpus, Cephalotaxus, Agathis, Araucaria, Taxus et Torreya.